(19) Europäisches Patentamt / European Patent Office / Office européen des brevets

(11) **EP 3 430 989 B1**

(12) **EUROPEAN PATENT SPECIFICATION**

(45) Date of publication and mention
of the grant of the patent:
**09.06.2021 Bulletin 2021/23**

(21) Application number: **17782507.2**

(22) Date of filing: **14.04.2017**

(51) Int Cl.:
*A61B 5/021* *(2006.01)*  *A61B 5/029* *(2006.01)*
*A61B 5/08* *(2006.01)*  *A61B 5/00* *(2006.01)*
*A61B 5/022* *(2006.01)*  *A61B 5/11* *(2006.01)*
*A61B 5/145* *(2006.01)*

(86) International application number:
**PCT/JP2017/015275**

(87) International publication number:
**WO 2017/179694 (19.10.2017 Gazette 2017/42)**

(54) **BIOLOGICAL INFORMATION ANALYSIS DEVICE, SYSTEM, PROGRAM, AND BIOLOGICAL INFORMATION ANALYSIS METHOD**

VORRICHTUNG, SYSTEM UND PROGRAMM ZUR ANALYSE BIOLOGISCHER INFORMATIONEN, VERFAHREN ZUR ANALYSE BIOLOGISCHER INFORMATIONEN

DISPOSITIF, SYSTÈME ET PROGRAMME D'ANALYSE D'INFORMATIONS BIOLOGIQUES, ET PROCÉDÉ D'ANALYSE D'INFORMATIONS BIOLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.04.2016 JP 2016082463**

(43) Date of publication of application:
**23.01.2019 Bulletin 2019/04**

(73) Proprietors:
• **Omron Corporation**
**Kyoto 600-8530 (JP)**
• **Omron Healthcare Co., Ltd.**
**Muko-shi, Kyoto 617-0002 (JP)**

(72) Inventors:
• **NAKAJIMA, Hiroshi**
**Kyoto-shi**
**Kyoto 600-8530 (JP)**
• **WADA, Hirotaka**
**Kyoto-shi**
**Kyoto 600-8530 (JP)**
• **TSUCHIYA, Naoki**
**Kyoto-shi**
**Kyoto 600-8530 (JP)**
• **KASAI, Masaaki**
**Kyoto-shi**
**Kyoto 600-8530 (JP)**

• **KAN, Eriko**
**Kyoto-shi**
**Kyoto 600-8530 (JP)**
• **UENOYAMA, Toru**
**Tokyo 108-0075 (JP)**
• **OBAYASHI, Keiichi**
**Kyoto-shi**
**Kyoto 600-8530 (JP)**
• **KOKUBO, Ayako**
**Kyoto-shi**
**Kyoto 600-8530 (JP)**
• **OTA, Yuya**
**Kyoto-shi**
**Kyoto 600-8530 (JP)**
• **SHIGA, Toshikazu**
**Muko-shi**
**Kyoto 617-0002 (JP)**
• **KUWABARA, Mitsuo**
**Muko-shi**
**Kyoto 617-0002 (JP)**
• **SATO, Hironori**
**Muko-shi**
**Kyoto 617-0002 (JP)**
• **MIYAGAWA, Ken**
**Muko-shi**
**Kyoto 617-0002 (JP)**
• **TSUTSUMI, Masakazu**
**Muko-shi**
**Kyoto 617-0002 (JP)**

**(Cont. next page)**

(74) Representative: **Isarpatent**
**Patent- und Rechtsanwälte Behnisch Barth**
**Charles**
**Hassa Peckmann & Partner mbB**
**Friedrichstrasse 31**
**80801 München (DE)**

(56) References cited:
**WO-A1-97/38626**     **WO-A2-2015/178439**
**JP-A- H08 317 912**     **JP-A- 2002 224 059**
**JP-A- 2004 223 271**     **JP-A- 2010 022 689**
**US-A1- 2008 064 965**     **US-A1- 2009 124 914**
**US-A1- 2010 228 139**

**Description**

TECHNICAL FIELD

The present invention relates to a technique for obtaining useful information from a measured blood pressure waveform.

RELATED ART

Techniques are known in which changes in the internal pressure of the radial artery are measured and the shape of pressure pulses (a blood pressure waveform) is recorded. Patent Document 1 (JP 2008-61824A) discloses measuring a blood pressure waveform through tonometry and obtaining information such as AI (Augmentation Index) value, pulse wave period, baseline fluctuation rate, sharpness, ET (Ejection Time), and the like from the blood pressure waveform. Patent Document 2 (JP 2005-532111A), meanwhile, discloses measuring a blood pressure waveform using a wristwatch-type blood pressure monitor, calculating a mean arterial pressure, a mean systolic pressure, a mean diastolic pressure, a mean systolic pressure index, and a mean diastolic pressure index from the blood pressure waveform, and then outputting an alert when those values deviate from reference values. Patent Document 3 (US 2010/228139 A1) discloses a living body inspection apparatus, wherein a pulse interval is obtained from a pulse wave signal, thereby performing a frequency analysis of the obtained pulse interval. Patent Document 4 (US 2009/124914 A1) discloses an analysis system and method for pulse diagnosis in Chinese medicine, which analyzes quantitatively the variances of pulse condition, wherein the analysis system for pulse diagnosis comprises a pulse signal collecting device to collect and generate blood pressure and electrocardiogram signals; and a signal process unit to receive and analyze the blood pressure signal series and electrocardiogram signal series. Patent Document 5 (US 2008/064965 A1) discloses methods and devices for measuring pulsus paradoxus, wherein the methods employ a combination of one or more forms of waveform analysis for the purpose of measuring pulsus paradoxus and diagnosing respiratory distress.

RELATED ART DOCUMENTS

PATENT DOCUMENTS

Patent Document 1: JP 2008-61824A
Patent Document 2: JP 2005-532111A
Patent Document 3 (US 2010/228139 A1)
Patent Document 4 (US 2009/124914 A1)
Patent Document 5 (US 2008/064965 A1)

SUMMARY OF THE INVENTION

PROBLEM TO BE SOLVED BY THE INVENTION

The inventors of the present invention are undertaking diligent research toward putting into practical use blood pressure measurement devices capable of accurately measuring a blood pressure waveform for each heartbeat during free movement. Through experiments using test subjects throughout the course of this development, the inventors of the present invention discovered that it may be possible to extract information pertaining to phenomena arising in a user's body by monitoring continuous blood pressure waveforms for each of heartbeats during free movement. For example, although it is naturally assumed that the user will move his/her body when measuring a blood pressure waveform during free movement, the user's body movement, changes in attitude, and so on may affect the blood pressure waveforms. Changes, abnormalities, and so on in the user's respiratory or circulatory state (e.g., the occurrence of apnea) may also affect the blood pressure waveforms. However, it is difficult to extract these types of information simply by evaluating the shape of the blood pressure waveform, changes over time, and so on.

Accordingly, an object of the present invention is to provide a novel technique for extracting useful information from blood pressure waveform time series data.

MEANS FOR SOLVING THE PROBLEMS

To achieve the above-described object, the present invention employs the following configurations.

The present invention provides a biological information analyzing device according to claim 1. When the time series data of the blood pressure waveform is converted into a frequency spectrum, features that cannot be known from the shapes of individual blood pressure waveforms (the original waveforms), changes over time therein, and so on sometimes appear. Thus according to the configuration of the present invention, information pertaining to blood pressure fluctuation (and particularly information that is difficult to detect simply by looking at the shapes of individual blood pressure waveforms, changes over time therein, and so on) can be extracted through relatively simple processing.

According to the invention, the indicator extraction unit extracts an indicator pertaining to the occurrence of body movement during blood pressure waveform measurement on the basis of the strength of the prescribed frequency component in the frequency spectrum. It has been confirmed that a prescribed frequency component in the frequency spectrum rises significantly when a given part of the body is moved during blood pressure waveform measurement. Accordingly, the strength of the prescribed frequency component can be used to detect body movement. At this time, it is preferable that the processing unit carry out a process of determining whether or not body movement has occurred during blood pressure waveform measurement on the basis of the indicator. Additionally, the processing unit may determine that body movement has occurred during blood pressure waveform measurement when the indicator is higher than a threshold. Through such processing, whether or not body movement has occurred during blood pressure waveform measurement can be determined easily. Information pertaining to whether or not body movement has occurred is extremely useful when using blood pressure waveform time series data.

According to the invention, the indicator extraction unit calculates the indicator for each of a plurality of body parts, and the frequency component used to calculate the indicator differs from body part to body part. Accordingly, whether or not body movement has occurred during blood pressure waveform measurement can be detected for each of a plurality of body parts.

Preferably, when the indicator is higher than the threshold, the processing unit carries out a process of reducing noise produced by body movement in the blood pressure waveform from the blood pressure waveform time series data. This makes it possible to improve the reliability of the blood pressure waveform time series data.

When the indicator is higher than the threshold, the processing unit may carry out a process of cutting or reducing a prescribed frequency component included in the blood pressure waveform time series data. This makes it possible to improve the reliability of the blood pressure waveform time series data.

When the indicator is higher than the threshold, the processing unit may carry out a process of discarding the blood pressure waveform time series data. This makes it possible to automatically discard blood pressure waveform time series data that may have a low reliability, which makes it possible to prevent low-reliability data from being used in later processes such as biological information analysis.

When the indicator is higher than the threshold, the processing unit may carry out a process of adding information indicating low reliability to the blood pressure waveform time series data. Adding such reliability information makes it possible to ensure the reliability of the blood pressure waveform time series data. As a result, low-reliability data can be prevented from being used in later processes such as biological information analysis. in an example, the indicator extraction unit may extract an indicator pertaining to respiratory and/or circulatory function of the user on the basis of the strength of the prescribed frequency component in the frequency spectrum. In another example, the processing unit may output information pertaining to respiratory and/or circulatory function of the user on the basis of the indicator. According to this configuration, information pertaining to respiratory or circulatory function can be obtained from the blood pressure waveform time series data itself.

For example, the inventors of the present invention discovered that a prescribed frequency component in the frequency spectrum rises significantly when apnea occurs during blood pressure waveform measurement. Accordingly, in another example, the indicator extraction unit may extract an indicator expressing an occurrence level of apnea as the indicator.

In another example, the processing unit may carry out a process of outputting a warning when the occurrence level of apnea is higher than a threshold. In another example, the indicator extraction unit may extract the indicator for different days, from blood pressure waveform time series data measured on different days, and the processing unit outputs information expressing a change over time in the respiratory and/or circulatory function of the user on the basis of the indicators for the different days. Outputting such information makes it possible to provide reference information pertaining to the occurrence of apnea to the user, a doctor, or the like.

The present invention provides a biological information analyzing system, a program, and a biological information analyzing method, according to claims 6, 7 and 8, respectively.

## EFFECTS OF THE INVENTION

According to the present invention, a technique for obtaining novel and useful information on the basis of blood pressure waveform data can be provided.

## BRIEF DESCRIPTION OF THE DRAWINGS

Fig. 1 is a diagram illustrating the external appearance of the overall configuration of a biological information analyzing system 10.

Fig. 2 is a block diagram illustrating the hardware configuration of the biological information analyzing system 10.

Fig. 3 is a cross-sectional view schematically illustrating the structure of a blood pressure measurement unit 20 and a measurement state.

Fig. 4 is a diagram illustrating a blood pressure waveform measured by the blood pressure measurement unit 20.

Fig. 5 is a block diagram illustrating processing performed by a biological information analyzing device 1.

Fig. 6 is a diagram illustrating the waveform of a pressure pulse (a blood pressure waveform) in the radial artery, for a single heartbeat.

Fig. 7 is a flowchart illustrating a body movement detection process according to Example 1.

Fig. 8 is a diagram illustrating an example of a blood pressure waveform frequency spectrum according to Example 1.

Fig. 9 is a diagram illustrating an example of a frequency spectrum of blood pressure waveform data in a set section after releasing a held breath, according to Example 2.

Fig. 10 is a flowchart illustrating a sleep apnea detection process according to Example 2.

Figs. 11A and 11B are diagrams illustrating display examples of analysis results according to Example 2.

EMBODIMENTS OF THE INVENTION

A preferred embodiment of the present invention will be described below with reference to the drawings. Note, however, that the descriptions of configurations given hereinafter should be changed as appropriate depending on the configuration of the device to which the invention is applied, various types of conditions, and so on, and the scope of the invention is not intended to be limited by the following descriptions.

Biological Information Analyzing System

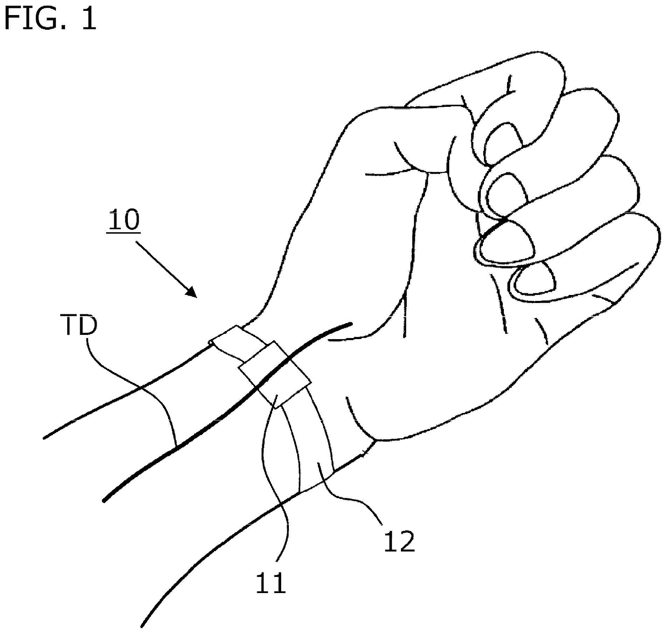

Fig. 1 is a diagram illustrating the external appearance of the overall configuration of a biological information analyzing system 10 according to an embodiment of the present invention. Fig. 1 illustrates a state in which the biological information analyzing system 10 is worn on the left wrist. The biological information analyzing system 10 includes a main unit 11 and a belt 12 fixed to the main unit 11. The biological information analyzing system 10 is what is known as a wearable device, and is worn so that the main unit 11 is in contact with the skin on the inner side of the wrist and so that the main unit 11 is arranged above a radial artery TD located beneath the skin. Although the present embodiment describes a configuration in which the device is worn above the radial artery TD, the configuration may be such that the device is worn above another superficial artery.

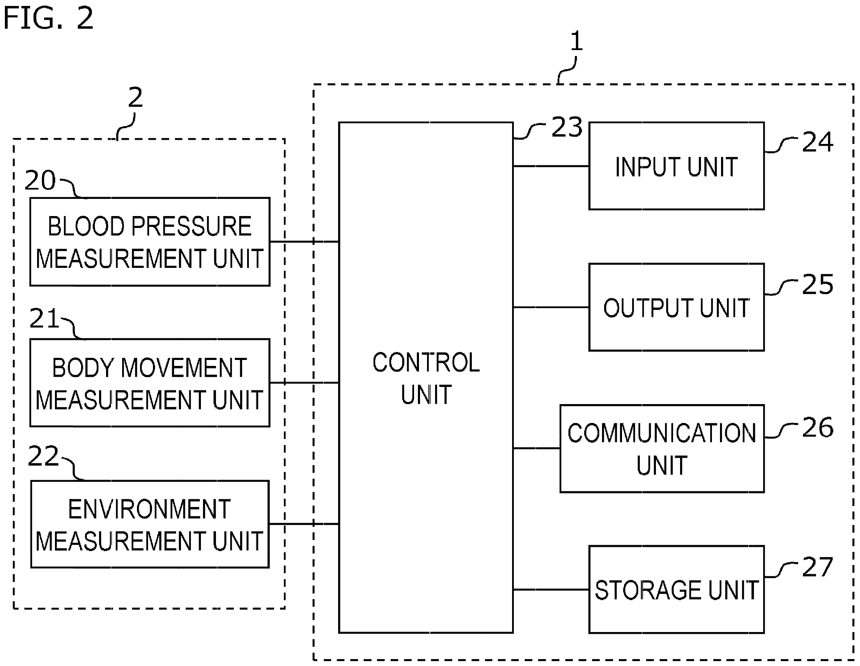

Fig. 2 is a block diagram illustrating the hardware configuration of the biological information analyzing system 10. Broadly speaking, the biological information analyzing system 10 includes a measurement unit 2 and a biological information analyzing device 1. The measurement unit 2 is a device that obtains information used to analyze biological information through measurement, and includes a blood pressure measurement unit 20, a body movement measurement unit 21, and an environment measurement unit 22. However, the configuration of the measurement unit 2 is not limited to that illustrated in Fig. 2. For example, units that measure biological information aside from blood pressure and body movement (body temperature, blood sugar, brain waves, and so on) may be added. Alternatively, units not used in the Example described later are not required configurations and may therefore be omitted from the biological information analyzing system 10. The biological information analyzing device 1 is a device that analyzes biological information on the basis of information obtained from the measurement unit 2, and includes a control unit 23, an input unit 24, an output unit 25, a communication unit 26, and a storage unit 27. The units 20 to 27 are connected to each other by a local bus and other signal lines so as to be capable of exchanging signals. The biological information analyzing system 10 also includes a power source (a battery), which is not illustrated.

The blood pressure measurement unit 20 is a unit that measures pressure pulses in the radial artery TD through tonometry. Tonometry is a non-invasive method of measuring pressure pulses using a pressure sensor, in which an artery is compressed from above the skin at an appropriate pressure to form a flat part in an artery TD, and the internal pressure and external pressure of the artery are balanced.

The body movement measurement unit 21 is a unit, including a three-axis accelerometer, that measures movement of a user's body (body movement) using the accelerometer. The body movement measurement unit 21 may include a circuit that converts the output of the three-axis accelerometer into a format that can be read by the control unit 23.

The environment measurement unit 22 is a unit that measures environment information that can affect the user's physical/mental state (and blood pressure in particular). The environment measurement unit 22 can include a temperature sensor, a humidity sensor, an illuminance sensor, an altitude sensor, a location sensor, and the like, for example. The environment measurement unit 22 may include a circuit that converts the output of these sensors into a format that can be read by the control unit 23.

The control unit 23 is a unit that handles a variety of processes, such as controlling the various parts of the biological information analyzing system 10, acquiring data from the measurement unit 2, storing the acquired data in the storage unit 27, processing/analyzing the data, inputting/outputting the data, and so on. The control unit 23 includes a hardware processor (called a CPU hereinafter), ROM (Read-Only Memory), RAM (Random Access Memory), and the like. The processing carried out by the control unit 23, which will be described later, is realized by the CPU reading programs stored in the ROM or the storage unit 27 and executing those programs. The RAM functions as a work memory when the control unit 23 carries out various types of processes. Although the present embodiment describes a configuration in which the control unit 23 acquires the data from the measurement unit 2 and stores the data in the storage unit 27, the configuration may be such that the data is stored (written) directly from the measurement unit 2 into the storage unit 27.

The constituent elements of the embodiment, e.g., the measurement units, an indicator extraction unit, a processing unit, a determination unit, a risk database, the input unit, the output unit, a case database, and the like may be provided as hardware in the biological information analyzing system 10. The indicator extraction unit, the processing unit, and the determination unit may receive executable programs stored in the storage unit 27 and execute those programs. The indicator extraction unit, the processing unit, and the determination unit may receive data from the blood pressure measurement unit 20, the body movement measurement unit 21, the environment measurement unit 22, the input unit 24, the output unit 25, the communication unit 26, the storage unit 27, and so on as necessary. Databases such as the risk database and the case database may be provided in the storage unit 27 or the like, and may store information arranged so that the data can be searched and accumulated with ease. The structure, operations, and the like of the biological information analyzing system 10 are disclosed in JP 2016-082069, for example. The structure, operations, and the like of the blood pressure measurement unit are disclosed in JP 2016-087003A.

The input unit 24 is a unit that provides an operation interface to the user. For example, operation buttons, switches, a touch panel, or the like can be used.

The output unit 25 is a unit that provides, to the user, an interface that outputs information. For example, a display device that outputs information as images (a liquid crystal display or the like), an audio output device or a buzzer that outputs information as audio, an LED that outputs information by emitting or extinguishing light, a vibration device that outputs information as vibrations, or the like can be used.

The communication unit 26 is a unit that carries out data communication with other devices. Any system, including wireless LAN, Bluetooth (registered trademark), or the like, may be used as the data communication system.

The storage unit 27 is a storage medium in which data can be stored and from which data can be read out, and stores programs executed by the control unit 23, measurement data obtained from the measurement units, various types of data obtained by processing the measurement data, and so on. The storage unit 27 is a medium that electrically, magnetically, optically, mechanically, or chemically stores the information to be stored. Flash memory can be used, for example. The storage unit 27 may be a portable type such as a memory card, or may be built into the biological information analyzing system 10.

Some or all of the body movement measurement unit 21, the environment measurement unit 22, the control unit 23, the input unit 24, the output unit 25, and the storage unit 27 may be configured as devices separate from the main unit 11. In other words, as long as the main unit 11 including the blood pressure measurement unit 20 and a circuit that controls the blood pressure measurement unit 20 can be worn on a wrist, the structures of other units can be designed as desired. In this case, the main unit 11 is linked to the other units via the communication unit 26. A variety of configurations are conceivable, such as implementing the functions of the control unit 23, the input unit 24, the output unit 25, and so on as a smartphone app, or obtaining necessary data from an activity meter having the functions of the body movement measurement unit 21, the environment measurement unit 22, and so on. Sensors that measure biological information aside from blood pressure may be provided as well. For example, a sleep sensor, a pulse oximeter (SpO2 sensor), a breathing sensor (flow sensor), a blood sugar level sensor, and so on may be combined as well.

Although the present embodiment describes providing a sensor that measures blood pressure (the blood pressure measurement unit 20) and a configuration that carries out analysis processes on blood pressure waveform data (the control unit 23 and the like) in a single device, these elements may be configured as separate entities. In the present embodiment, a configuration that carries out analysis processes on biological information (the control unit 23 and the like) is called a "biological information analyzing device", and a device configured by combining a measurement unit and the biological information analyzing device is called a "biological information analyzing system". However, these names are used for the sake of simplicity, and the measurement unit and the configuration that carries out analysis processes on biological information may as a whole be called a "biological information analyzing device", or other names may be used instead.

Blood Pressure Waveform Measurement

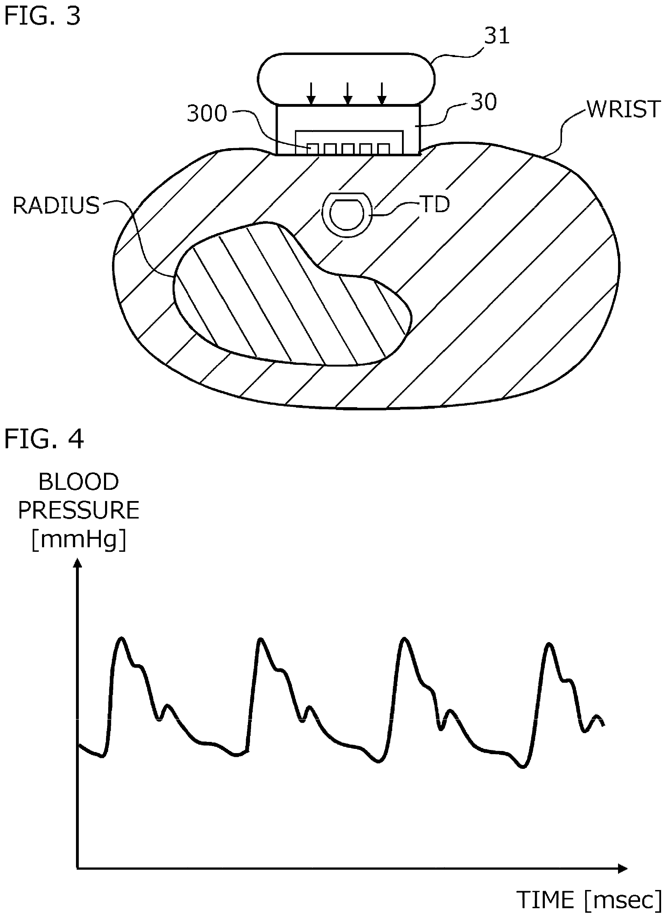

Fig. 3 is a cross-sectional view schematically illustrating the structure of the blood pressure measurement unit 20 and a measurement state. The blood pressure measurement unit 20 includes a pressure sensor 30, and a compression mechanism 31 for pressing the pressure sensor 30 against the wrist. The pressure sensor 30 includes a plurality of pressure detection elements 300. The pressure detection elements 300 are elements that detect a pressure and convert the pressure into an electrical signal, and elements that use a piezoresistance effect, for example, can be favorably used. The compression mechanism 31 is constituted by, for example, an air bladder and a pump that adjusts the internal pressure of the air bladder. When the control unit 23 controls the pump to increase the internal pressure of the air bladder, the air bladder expands and presses the pressure sensor 30 against the surface of the skin. Note that the compression mechanism 31 may be any mechanism capable of adjusting a compressive force of the pressure sensor 30 against the surface of the skin, and is not limited to a mechanism employing an air bladder.

When the biological information analyzing system 10 is secured to the wrist and started, the control unit 23 controls the compression mechanism 31 of the blood pressure measurement unit 20 to keep the compressive force of the pressure sensor 30 in an appropriate state (a tonometry state). Pressure signals detected by the pressure sensor 30 are then acquired sequentially by the control unit 23. The pressure signals obtained by the pressure sensor 30 are generated by taking analog physical amounts (e.g., voltage values) outputted by the pressure detection elements 300 and digitizing those physical amounts through an A/D conversion circuit or the like that employs a known technique. Suitable analog values such as current values, resistance values, or the like may be employed as the analog physical amounts, depending on the types of the pressure detection elements 300. The signal processing such as A/D conversion may be carried out by providing a predetermined circuit in the blood pressure measurement unit 20, or may be carried out by another unit (not shown) provided between the blood pressure measurement unit 20 and the control unit 23. The pressure signals acquired by the control unit 23 correspond to instantaneous values of the internal pressure of the radial artery TD. Accordingly, time series data of a blood pressure waveform can be obtained by acquiring a pressure signal at a time granularity and continuity that enable the blood pressure waveform of a single heartbeat to be obtained. The control unit 23 stores the pressure signals sequentially obtained from the pressure sensor 30 in the storage unit 27 along with information of the measurement times of the signals. The control unit 23 may store the acquired pressure signals as-is in the storage unit 27, or may store the pressure signals in the storage unit 27 after applying necessary signal processing to the pressure signals. The "necessary signal processing" may include processing for correcting the pressure signals so that the amplitude of the pressure signals matches a blood pressure value (e.g., an upper arm blood pressure), processing for reducing or removing noise from the pressure signals, or the like, for example.

Fig. 4 is a diagram illustrating a blood pressure waveform measured by the blood pressure measurement unit 20. The horizontal axis represents time, and the vertical axis represents blood pressure. The sampling frequency can be set as desired, but is preferably set to greater than or equal to 100 Hz in order to reproduce the shape characteristics of the waveform of a single heartbeat. Because the period of a single heartbeat is approximately one second, approximately 100 or more data points can be acquired in the waveform of a single heartbeat.

The blood pressure measurement unit 20 according to the present embodiment has advantages such as those described below.

A blood pressure waveform can be measured for each heartbeat. Thus for example, a variety of indicators related to blood pressure, heart condition, cardiovascular risk, and so on can be obtained on the basis of the shape characteristics of the blood pressure waveform. Additionally, the instantaneous value of the blood pressure can be monitored, which makes it possible to immediately detect blood pressure surges (sudden rises in blood pressure value), reliably detect blood pressure fluctuations and disturbances in the blood pressure waveform appearing only over extremely short amounts of time (one to several heartbeats), and so on.

Blood pressure monitors that are secured to the wrist or upper arm and measure blood pressure through the oscillometric method are in practical use as portable blood pressure monitors. However, a conventional portable blood pressure monitor can only measure a mean blood pressure value from fluctuations in the internal pressure of a cuff over several heartbeats, spanning several seconds to several tens of seconds, and thus cannot obtain time series data of a blood pressure waveform for each heartbeat, as is the case with the blood pressure measurement unit 20 according to the present embodiment.

The blood pressure waveform time series data can be recorded. When the blood pressure waveform time series data is acquired, a variety of indicators pertaining to blood pressure, heart condition, cardiovascular risk, and the like can be obtained by finding characteristics pertaining to changes in the blood pressure waveform over time, analyzing the frequency of the time series data and extracting specific frequency components, and so on, for example.

Because the device is configured as a portable (wearable) device, measurements place little burden on the user, and it is relatively easy to take continuous measurements for long periods of time, monitor blood pressure throughout the entire day, and so on. Furthermore, the portable form makes it possible not only to measure resting blood pressure, but also to measure changes in blood pressure during free movement (e.g., during daily activities, exercise, and so on). This in turn makes it possible to understand the effects of daily activities (sleeping, meals, commuting, work, taking medicine, and so on), exercise, and so on on blood pressure, for example.

Conventional products are devices of a type in which a blood pressure measurement unit is secured to the arm or wrist and the measurement is taken in a state of rest, and changes in blood pressure during daily activities, exercise, and so on cannot be measured, as with the biological information analyzing system 10 according to the present embodiment.

It is easy to link or combine the system with other sensors. For example, causal relationship evaluations or compound evaluations can be made using information obtained from other sensors (body movement, environment information such as temperature, other biological information such as SpO2 or breathing, and the like).

Biological Information Analyzing Device

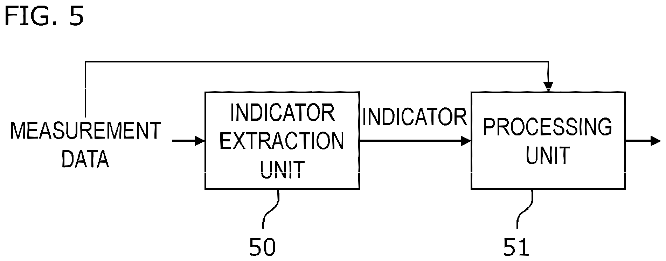

Fig. 5 is a block diagram illustrating processing performed by the biological information analyzing device 1. As illustrated in Fig. 5, the biological information analyzing device 1 includes an indicator extraction unit 50 and a processing unit 51. In the present embodiment, the processes of the indicator extraction unit 50 and the processing unit 51 may be realized by the control unit 23 executing necessary programs. These programs may be stored in the storage unit 27. When executing the necessary programs, the control unit 23 loads the programs in question, which are stored in the ROM or the storage unit 27, into the RAM. The control unit 23 then uses a CPU to interpret and execute the programs loaded into the RAM, and controls the various constituent elements. However, some or all the processing of the indicator extraction unit 50 and the processing unit 51 may be implemented by a circuit such as an ASIC, a FPGA, or the like. Alternatively, some or all of the processing of the indicator extraction unit 50 and the processing unit 51 may be implemented by a computer separate from the main unit 11 (e.g., a smartphone, a tablet terminal, a personal computer, a cloud server, or the like).

The indicator extraction unit 50 obtains, from the storage unit 27, the blood pressure waveform time series data measured continuously by the blood pressure measurement unit 20. The indicator extraction unit 50 extracts an indicator pertaining to characteristics of the blood pressure waveform, from the obtained blood pressure waveform time series data. Here, "characteristics of the blood pressure waveform" include shape characteristics of the blood pressure waveform for a single heartbeat, changes in the blood pressure waveform over time, a frequency component of the blood pressure waveform, and so on. The blood pressure waveform characteristics are not limited thereto, however. The extracted indicator is output to the processing unit 51. Because there are a variety of blood pressure waveform characteristics and indicators, the characteristics and indicators to be extracted can be designed and selected as appropriate in accordance with the purpose of the processing by the processing unit 51. The characteristics and indicators that can be extracted from the blood pressure waveform measurement data in the present embodiment will be described later.

When finding the indicator, the indicator extraction unit 50 can use the measurement data from the body movement measurement unit 21 and/or the measurement data from the environment measurement unit 22 in addition to the blood pressure waveform measurement data. Although not illustrated, measurement data from a sleep sensor, an SpO2 sensor, a breathing sensor (flow sensor), a blood sugar level sensor, or the like may be combined as well. Carrying out a compound analysis on multiple types of measurement data obtained from multiple types of sensors enables a higher level of information analysis to be carried out on the blood pressure waveform. For example, the blood pressure waveform data can be classified into user states, such as resting and active, times of high and low temperature, times of light and deep sleep, breathing and apnea, and so on. Alternatively, causal relationships, correlations, and so on among the measurement data can be evaluated, by extracting the effects of body movement, activity amounts and activity intensity, changes in temperature, how breathing or apnea manifests, and so on on blood pressure. Note that apnea includes obstructive apnea, central apnea, and mixed apnea.

The processing unit 51 receives the indicator extracted by the indicator extraction unit 50. The processing unit 51 carries out processing on the basis of the received indicator. A variety of processes are conceivable as the processes based on the indicator. For example, values of or changes in the extracted indicator may be provided to the user, a doctor, a nurse, or the like and used for health management, treatment, health guidance, and so on.

Information Obtained from Blood Pressure Waveform

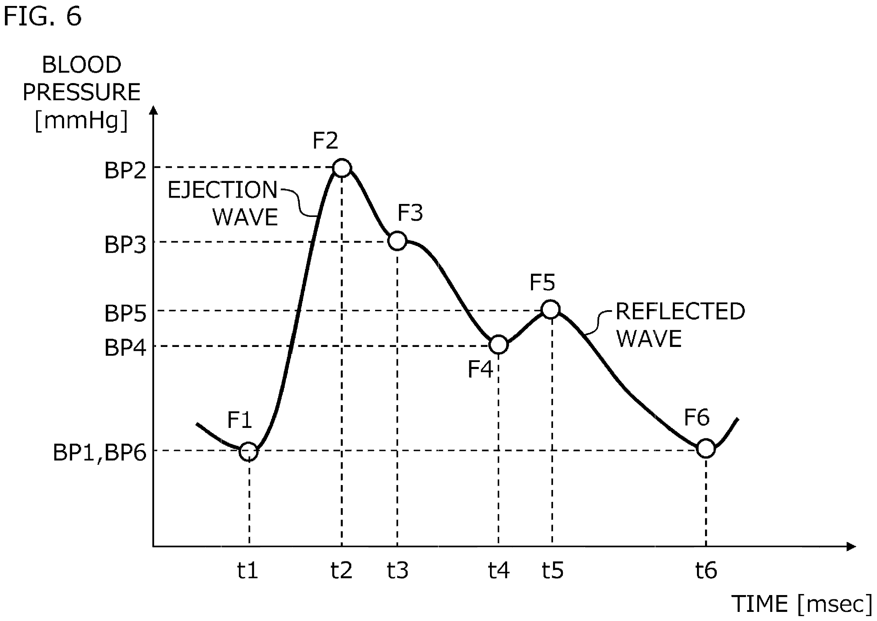

Fig. 6 is a diagram illustrating the waveform of a pressure pulse (a blood pressure waveform) in the radial artery, for a single heartbeat. The horizontal axis represents time t [msec], and the vertical axis represents blood pressure BP [mmHg].

The blood pressure waveform is a compound wave including an "ejection wave" produced when the heart contracts to expel blood and a "reflected wave" produced when the ejection wave is reflected by peripheral vessels, arterial branches, and so on. Examples of characteristic points that can be extracted from a blood pressure waveform corresponding to a single heartbeat are listed below.

· Point F1 is a point corresponding to the rise of the pressure pulse. The point F1 corresponds to an ejection start point of the heart, i.e., a point when the aortic valve opens.
· Point F2 is a point where the amplitude (pressure) of the ejection wave is maximum (a first peak).
· Point F3 is an inflection point appearing partway along the fall of the ejection wave due to the superposition of the reflected wave.
· Point F4 is a minimum point appearing between the ejection wave and the reflected wave, and is also called a "notch". This corresponds to a point when the aortic valve closes.
· Point F5 is a peak in the reflected wave appearing after point F4 (a second peak).
· Point F6 is the end point of the single heartbeat, and corresponds to the ejection start point of the next heartbeat, i.e., the starting point of the next heartbeat.

The indicator extraction unit 50 may use any algorithm to detect the characteristic points. For example, the indicator extraction unit 50 may extract a characteristic point (inflection point) of the blood pressure waveform by operating so as to find a nth-order differential waveforms of the blood pressure waveform and detect a zero crossing point thereof (for points F1, F2, F4, F5, and F6, this can be detected from a first-order differential waveform, and for point F3, from a second-order or fourth-order waveform). Alternatively, the indicator extraction unit 50 may identify the positions of the characteristic points by reading out a waveform pattern, in which characteristic points have been arranged in advance, from the storage unit 27, and fitting the blood pressure waveform in question to the waveform pattern.

By operating on the basis of the times t and the pressures BP of the above characteristic points F1 to F6, the indicator extraction unit 50 can obtain a variety of information (values, feature amounts, indicators, and the like) from the blood pressure waveform of a single heartbeat. The following provides representative examples of information that can be obtained from the blood pressure waveform. Note that tx and BPx represent the time and blood pressure, respectively, of a characteristic point Fx.

- pulse wave interval (heartbeat period) $TA = t6 - t1$
- heart rate $PR = 1/TA$
- pulse wave rise time $UT = t2 - t1$
- systole $TS = t4 - t1$
- diastole $TD = t6 - t4$
- reflected wave delay time $= t3 - t1$
- maximum blood pressure (systolic blood pressure) $SBP = BP2$
- minimum blood pressure (diastolic blood pressure) $DBP = BP1$
- mean blood pressure MAP = area of blood pressure waveform from t1 to t6 / heartbeat period TA
- systolic mean blood pressure = area of blood pressure waveform from t1 to t4 / systole TS
- diastolic mean blood pressure = area of blood pressure waveform from t4 to t6 / diastole TD
- pulse pressure PP = maximum blood pressure SBP - minimum blood pressure DBP
- late systolic pressure $SBP2 = BP3$
- AI (Augmentation Index) = (late systolic pressure SBP2 - minimum blood pressure DBP) / pulse pressure PP

Basic statistical amounts of this information (values, feature amounts, indicators) can also be used as indicators. The basic statistical amounts include representative values (mean values, median values, mode values, maximum values, minimum values, and the like) and dispersion (variance, standard deviation, coefficient of variation, and the like), for example. Changes over time in this information (values, characteristic values, indicators) can also be used as indicators.

By computing a plurality of pieces of beat information, the indicator extraction unit 50 can obtain an indicator called BRS (baroreflex sensitivity). This is an indicator expressing the capacity of blood pressure to regulate to a constant value. The spontaneous sequence method is an example of the calculation method. This is a method in which only a sequence in which the maximum blood pressure SBP and the pulse wave interval TA rise or fall in synchronization for three or more consecutive beats is extracted, the maximum blood pressure SBP and the pulse wave interval TA are plotted on a two-dimensional plane, and a slope obtained when a regression line is found through the least-squares method is defined as the BRS.

As described thus far, using the biological information analyzing system 10 according to the present embodiment makes it possible to obtain a variety of information from the blood pressure waveform data. However, the biological information analyzing system 10 need not include functions for obtaining all of the above-described information. It is acceptable to provide only the functions for obtaining the necessary information, in accordance with the configuration, user, purpose of usage, location of usage, and so on of the biological information analyzing system 10. Additionally, the configuration may be such that the functions are provided as program modules (application software), and functions can be added by installing the required program modules in the biological information analyzing system 10.

The following Examples describe specific examples of the application of the biological information analyzing system 10.

Example 1

This Example is an example in which the user's body movement during measurement is detected using blood pressure waveform time series data.

Conventional blood pressure monitors using the tonometry method assume that measurement is carried out in a state of rest, and do not assume that the user will move during measurement. However, a portable device such as that described in this Example must assume that the user may move during measurement. User movement during measurement produces noise, which appears in the blood pressure waveform. Thus when detecting the user's body movement, processes such as discarding blood pressure waveform data measured at the time of movement (due to poor reliability), reducing noise produced by the user's body movement (noise removal or correcting the blood pressure waveform data), adding metadata indicating low reliability to the blood pressure waveform data, and so on are effective. Specific configurations and processes will be described hereinafter.

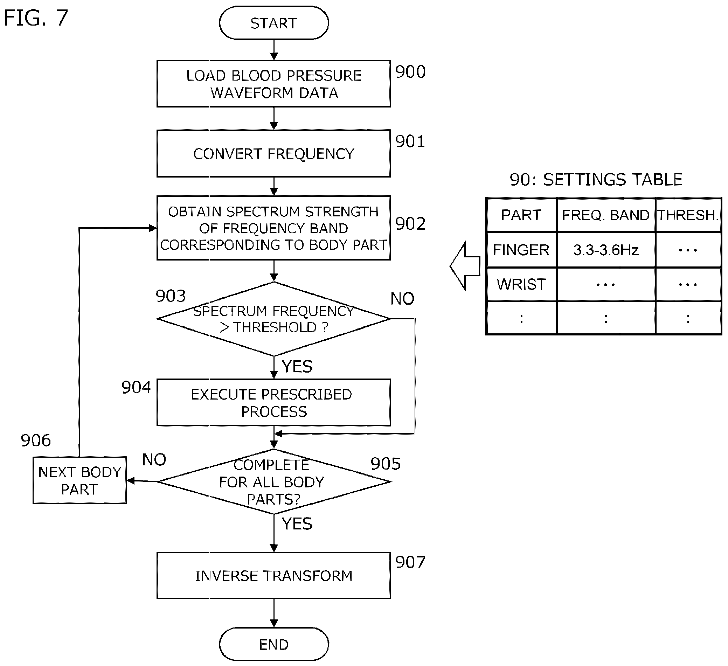

Fig. 7 illustrates an example of a flowchart of a body movement detection process according to this Example. First, the indicator extraction unit 50 loads the blood pressure waveform data from the storage unit 27 (step 900). For example, several minutes' to several hours' worth of time series data is loaded. The indicator extraction unit 50 subjects the blood pressure waveform data to frequency conversion (e.g., a fast Fourier transform) to obtain frequency spectrum data of the blood pressure waveform (step 901).

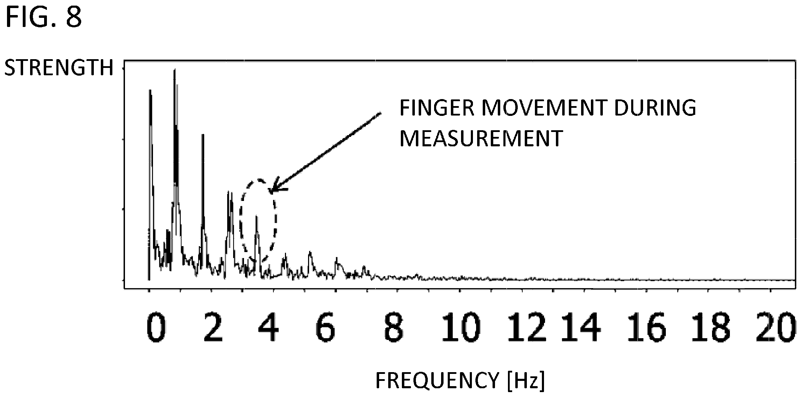

Fig. 8 illustrates an example of the blood pressure waveform frequency spectrum. The horizontal axis represents frequency, and the vertical axis represents strength (amplitude). Here, it can be seen that a clear spectrum peak appears in a frequency band of approximately 3.3 to 3.6 Hz. Through experimentation, the inventors of the present invention found trends such as the following:

(1) A prescribed frequency component rises significantly when the user moves his/her body during measurement.
(2) Which frequency band component rises depends on the part that is moved (Fig. 8 illustrates an example where a finger has been moved).

Accordingly, the indicator extraction unit 50 refers to a settings table 90 in which "body parts", "frequency bands", and "thresholds" are associated with each other, and calculates a spectral strength of the "frequency band" corresponding to each "body part" (step 902). The spectral strength of this specific frequency band can be used as an indicator pertaining to the occurrence of body movement during measurement. Although the settings table 90 is stored in the storage unit 27 in this Example, the settings table 90 may be present in storage, a server, or the like external to the biological information analyzing device 1 (e.g., a cloud server, online storage, or the like).

The processing unit 51 compares the spectral strength calculated in step 902 with the "threshold" (step 903). If the spectral strength is greater than the threshold, the processing unit 51 determines that that body part has moved during the measurement, and executes a prescribed process (step 904).

The "prescribed process" is a process such as discarding the blood pressure waveform data, reducing noise caused by the user's body movement (noise removal or correcting the blood pressure waveform data), adding metadata indicating low reliability to the blood pressure waveform data, or the like. Which of these processes to execute may be set by the user. The processing unit 51 may instead execute a plurality of processes. A filtering process can be given as an example of an effective method for removing noise or correcting the blood pressure waveform data. For example, with a body part for which a body movement component appears in a frequency band of approximately 3.3 to 3.6 Hz as illustrated in Fig. 8, a filter (high-pass, low-pass, bandpass, or the like) that cuts or reduces a frequency component of approximately 3.3 to 3.6 Hz may be used.

The indicator extraction unit 50 repeats the processes of steps 902 to 904 while varying the frequency band (frequency component) used to calculate the indicator from body part to body part in accordance with the settings in the settings table 90 (steps 905, 906). As a result, indicators pertaining to body movement during measurement are calculated for each body part, and necessary processing such as noise reduction is executed by the processing unit 51 when it is determined that there is body movement. The processing unit 51 then subjects the frequency spectrum to an inverse transform to obtain corrected blood pressure waveform data (step 907).

According to the configuration described thus far, body movement during measurement can be detected on the basis of the blood pressure waveform time series data. Additionally, when body movement has been detected, countermeasures such as discarding the blood pressure waveform data, reducing noise, adding reliability information, and so on can be carried out automatically, which makes it possible to improve the reliability of the blood pressure waveform data. The occurrence of body movement may be detected using information obtained by the body movement measurement unit 21. However, the body movement measurement unit 21 cannot detect movement in the fingers, movement of the wrist on the side where the body movement measurement unit 21 is not worn, and so on. However, body movement detection using blood pressure waveform data has an advantage in that there are fewer blind spots (movements that cannot be detected).

In this Example, the configuration is such that a filtering process or the like is carried out only when the spectral strength of the prescribed frequency component is greater than the threshold (i.e., when it is determined that there is body movement). As a variation, the configuration may be such that a filtering process is always carried out on frequency bands in which a body movement component can appear, regardless of whether or not the spectral strength is greater than the threshold (i.e., without carrying out a process for comparing the spectral strength with a threshold), for example. However, applying the filter uniformly even when there is no body movement may result in the loss even of necessary information (blood pressure waveform signal components). For this reason, it is preferable that a filter be applied only when it is determined that there is body movement, as in this Example.

Example 2

This Example is an example in which the occurrence of apnea is detected using blood pressure waveform time series data.

A PSG test is typically carried out to determine sleep apnea syndrome. However, PSG tests are expensive, and because the testing must be carried out with the subject sleeping over at a medical institution, PSG tests take a long time. Furthermore, sleep states are recorded by attaching electrodes to the necessary parts of the body, such as the head and face, with tape, which is uncomfortable for and places a large burden on the subject.

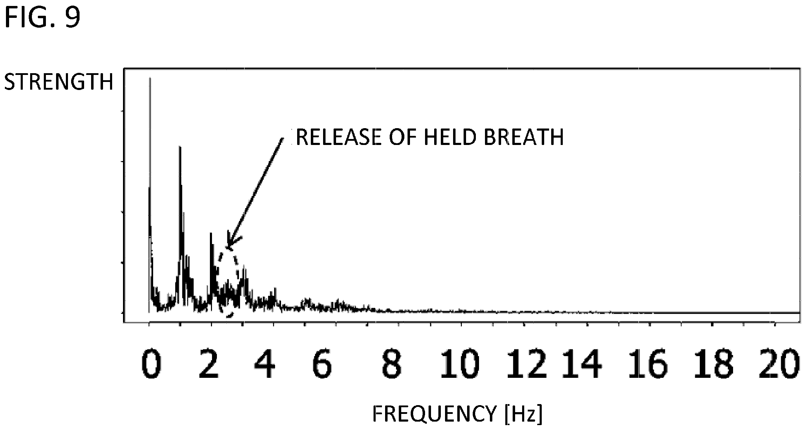

The inventors of the present invention carried out Valsalva tests, which correspond to a state of apnea, in subject experiments, and obtained results such as those illustrated in Fig. 9 by carrying out frequency analysis on blood pressure waveform data in a set section after releasing a held breath. Fig. 9 illustrates a frequency spectrum of blood pressure waveform data in a set section after releasing a held breath (corresponding to a state immediately after recovering from apnea). A peak in a frequency band of approximately 2.2 to 3.0 Hz was confirmed in the blood pressure waveform data of the set section after releasing a held breath. Such peaks can be assumed to indicate the possibility of a frequency component produced by holding the breath, i.e., caused by the occurrence of apnea. On the basis of this knowledge, a method for easily confirming the extent and trend of apnea (called an "apnea occurrence level" hereinafter) from blood pressure waveform time series data will be proposed.

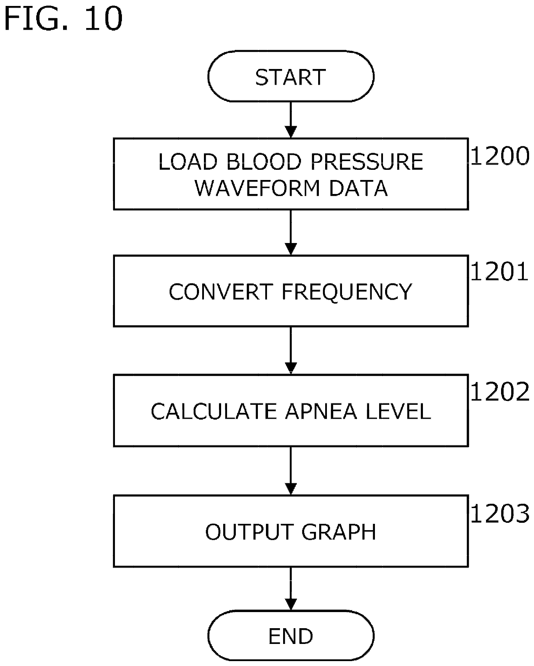

Fig. 10 illustrates an example of a flowchart of an apnea detection process according to this Example. First, the indicator extraction unit 50 loads the blood pressure waveform data from the storage unit 27 (step 1200). For example, several tens of minutes' to several hours' (or one night's) worth of time series data is loaded. The indicator extraction unit 50 subjects the blood pressure waveform data to frequency conversion (e.g., a fast Fourier transform) to obtain frequency spectrum data of the blood pressure waveform (step 1201).

Next, through the following equation, the indicator extraction unit 50 calculates a total value S of a spectrum X(h) of frequency band [hmin, hmax] (where hmin = 2.2 Hz and hmax = 3.0 Hz, for example) in which a frequency component caused by the occurrence of apnea appears, and then finds an apnea level L on the basis of the magnitude of S (step 1202). The apnea level L is a value that increases with the frequency component in the aforementioned frequency band, and an indicator expressing the occurrence level of apnea can be used.

## [Equation 1]

$$S = \sum_{h\min}^{h\max} X(h)$$

$$L = \begin{cases} 0 & (S < Th0) \\ 1 & (Th0 \leq S < Th1) \\ 2 & (Th1 \leq S < Th2) \\ 3 & (Th2 \leq S) \end{cases}$$

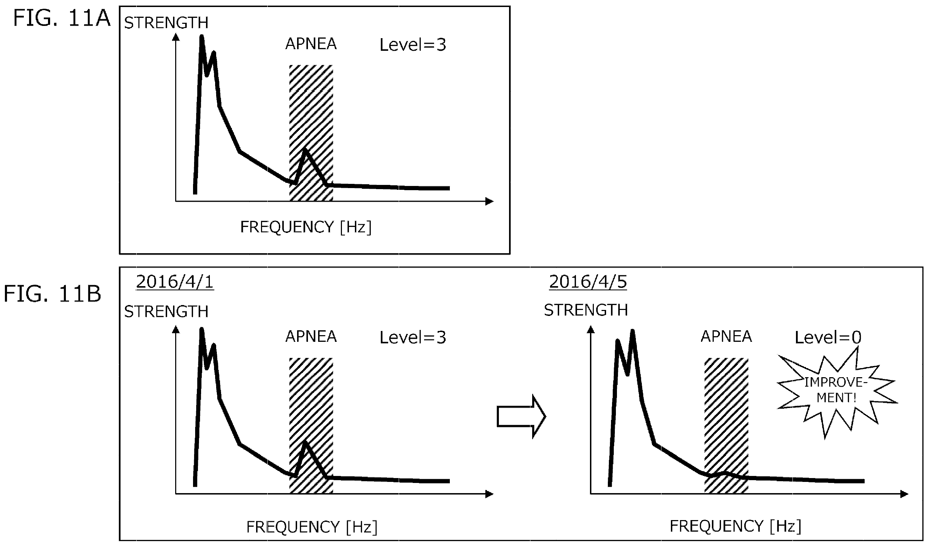

Then, the processing unit 51 outputs a blood pressure waveform frequency analysis result (a spectrum graph) and the apnea level L to a display device or a printer (step 1203). Figs. 11A and 11B are examples of the display of the analysis result. In the display example illustrated in Fig. 11A, the frequency spectrum of the blood pressure waveform time series data for a prescribed period (horizontal axis: frequency; vertical axis: strength) and the value of the apnea level L are displayed. To facilitate understanding, the frequency band in which the frequency component caused by the occurrence of apnea is hatched. On the other hand, changes over time in the apnea level L are indicated in the display example illustrated in Fig. 11B. Information such as that illustrated in Fig. 11B can be provided by, for example, the indicator extraction unit 50 carrying out frequency analysis on the blood pressure waveform time series data measured on different days (April 1, 2016 and April 5, 2016), and then calculating the frequency spectrum and apnea level L for each of those days. If the apnea level L has decreased over time, the processing unit 51 may output an indication that the apnea state has improved, whereas if the reverse is true, the processing unit 51 may output an indication that the apnea state has worsened. By visualizing the blood pressure waveform frequency analysis result in this manner and presenting the result to the user him/her self, a doctor, or the like, the extent of and trends in the occurrence of apnea can be confirmed with ease. Additionally, comparing with past analysis results makes it possible to confirm whether the state is improving or worsening, ascertain the effects of medicine, and so on.

The processing unit 51 may output a warning if the user's apnea level L is greater than or equal to 1. Furthermore, the apnea detection process according to this Example may be carried out at prescribed intervals of time (e.g., once every five minutes) while the user is sleeping, and a warning may be output to the user when the occurrence of apnea is detected. This makes it possible to ascertain dangerous time spans where apnea occurs.

Although this Example describes calculating an indicator expressing the occurrence level of apnea as an indicator pertaining to respiratory function, another indicator pertaining to respiratory function, an indicator pertaining to circulatory function, or the like may instead be calculated through the same type of method. Additionally, although this Example describes an example of outputting a warning when the indicator exceeds a threshold, if a lower indicator represents a higher risk with respect to respiratory or circulatory function, the warning may be output when the indicator drops below a threshold.

INDEX TO THE REFERENCE NUMERALS

| | |
|---|---|
| 1... | biological information analyzing device |
| 2... | measurement unit |
| 10... | biological information analyzing system |
| 11... | main unit |
| 12... | belt |
| 20... | blood pressure measurement unit |
| 21... | body movement measurement unit |
| 22... | environment measurement unit |
| 23... | control unit |
| 24... | input unit |
| 25... | output unit |
| 26... | communication unit |
| 27... | storage unit |

30...      pressure sensor
31...      compression mechanism
300...     pressure detection element
50...      indicator extraction unit
51...      processing unit
90...      settings table

**Claims**

1.  A biological information analyzing device (1) comprising:

    an indicator extraction unit (50) configured to convert, into a frequency spectrum, time series data of a blood pressure waveform continuously and non-invasively measured by a sensor (30) for each of heartbeats, and extract an indicator based on a strength of a prescribed frequency component in the frequency spectrum; and
    a processing unit (51) configured to carry out a process based on the extracted indicator,
    wherein the indicator extraction unit (50) is configured to extract an indicator pertaining to the occurrence of body movement during blood pressure waveform measurement on the basis of the strength of the prescribed frequency component in the frequency spectrum, and to calculate the indicator for each of a plurality of body parts; and
    wherein the frequency component used to calculate the indicator differs from body part to body part.

2.  The biological information analyzing device (1) according to claim 1,
    wherein the processing unit (51) is configured to carry out a process of determining whether or not body movement has occurred during blood pressure waveform measurement on the basis of the indicator.

3.  The biological information analyzing device (1) according to claim 2,
    wherein the processing unit (51) is configured to determine that body movement has occurred during blood pressure waveform measurement when the indicator is higher than a threshold.

4.  The biological information analyzing device (1) according to any one of claims 1 to 3,
    wherein the processing unit (51) is configured to carry out a process of reducing noise produced by body movement in the blood pressure waveform from the blood pressure waveform time series data when the indicator is higher than the threshold.

5.  The biological information analyzing device (1) according to any one of claims 1 to 3,
    wherein the processing unit (51) is configured to carry out when the indicator is higher than the threshold:

    a process of cutting or reducing a prescribed frequency component included in the blood pressure waveform time series data,
    a process of discarding the blood pressure waveform time series data, or,
    a process of adding information indicating low reliability to the blood pressure waveform time series data.

6.  A biological information analyzing system (10) comprising:

    a sensor (30), which is configured to non-invasively measure a blood pressure waveform for each of heartbeats; and
    the biological information analyzing device (1) according to any one of claims 1 to 5, the biological information analyzing device (1) is configured to analyze biological information using data of the blood pressure waveform measured continuously by the sensor (30).

7.  A program causing a processor to function as the indicator extraction unit (50) and the processing unit (51) of the biological information analyzing device (1) according to any one of claims 1 to 5.

8.  A biological information analyzing method comprising:

    a step of converting, by an indicator extraction unit (50), into a frequency spectrum, time series data of a blood pressure waveform continuously measured by a sensor (30) that is worn on a user's body and can non-invasively measure the blood pressure waveform for each of heartbeats, and extracting an indicator based on a strength of a prescribed frequency component in the frequency spectrum; and
    a step of carrying out a process based on the extracted indicator by a processing unit (51),
    wherein an indicator pertaining to the occurrence of body movement during blood pressure waveform measurement on the basis of the strength of the prescribed frequency component in the frequency spectrum is extracted by the indicator extraction unit (50), and the indicator is calculated by the indicator extraction unit (50) for each of a plurality of body parts; and
    wherein the frequency component used to calculate the indicator differs from body part to body part.

**Patentansprüche**

1. Vorrichtung zur Analyse biologischer Informationen (1), umfassend:

    eine Indikatorableitungseinheit (50), die so eingerichtet ist, dass sie Zeitreihendaten einer Blutdruck-Wellenform, die kontinuierlich und nicht invasiv von einem Sensor (30) für jeden der Herzschläge gemessen wird, in ein Frequenzspektrum umwandelt und auf Grundlage einer Stärke einer festgesetzten Frequenzkomponente im Frequenzspektrum einen Indikator ableitet; und
    eine Verarbeitungseinheit (51), die so eingerichtet ist, dass sie auf Grundlage des abgeleiteten Indikators einen Vorgang durchführt,
    wobei die Indikatorableitungseinheit (50) so eingerichtet ist, dass sie einen Indikator, der zum Auftreten einer Körperbewegung während der Messung der Blutdruck-Wellenform gehört, auf Grundlage der Stärke der festgesetzten Frequenzkomponente im Frequenzspektrum ableitet und den Indikator für jeden von einer Vielzahl von Körperteilen berechnet; und
    wobei sich die zum Berechnen des Indikators eingesetzte Frequenzkomponente von Körperteil zu Körperteil unterscheidet.

2. Vorrichtung zur Analyse biologischer Informationen (1) nach Anspruch 1,
    wobei die Verarbeitungseinheit (51) so eingerichtet ist, dass sie einen Vorgang durchführt, um auf Grundlage des Indikators festzustellen, ob während der Messung der Blutdruck-Wellenform eine Körperbewegung stattgefunden oder nicht stattgefunden hat.

3. Vorrichtung zur Analyse biologischer Informationen (1) nach Anspruch 2,
    wobei die Verarbeitungseinheit (51) so eingerichtet ist, dass sie feststellt, dass während der Messung der Blutdruck-Wellenform eine Körperbewegung stattgefunden hat, wenn der Indikator über einem Schwellenwert liegt.

4. Vorrichtung zur Analyse biologischer Informationen (1) nach einem der Ansprüche 1 bis 3,
    wobei die Verarbeitungseinheit (51) so eingerichtet ist, dass sie einen Vorgang zum Verringern von Rauschen, das durch eine Körperbewegung in der Blutdruck-Wellenform erzeugt wird, in den Zeitreihendaten einer Blutdruck-Wellenform durchführt, wenn der Indikator über dem Schwellenwert liegt.

5. Vorrichtung zur Analyse biologischer Informationen (1) nach einem der Ansprüche 1 bis 3,
    wobei die Verarbeitungseinheit (51) so eingerichtet ist, dass sie, wenn der Indikator über dem Schwellenwert liegt:

    einen Vorgang zum Abschneiden oder Vermindern einer festgesetzten Frequenzkomponente durchführt, die in den Zeitreihendaten einer Blutdruck-Wellenform enthalten ist,
    einen Vorgang zum Verwerfen der Zeitreihendaten einer Blutdruck-Wellenform durchführt oder
    einen Vorgang zum Hinzufügen von Informationen, die eine geringe Zuverlässigkeit angeben, zu den Zeitreihendaten einer Blutdruck-Wellenform durchführt.

6. System zur Analyse biologischer Informationen (10), umfassend:

    einen Sensor (30), der so eingerichtet ist, dass er nicht invasiv eine Blutdruck-Wellenform für jeden der Herzschläge misst; und
    die Vorrichtung zur Analyse biologischer Informationen (1) nach einem der Ansprüche 1 bis 5, wobei die Vorrichtung zur Analyse biologischer Informationen (1) so eingerichtet ist, dass sie unter Verwendung von Daten zur Blutdruck-Wellenform, die kontinuierlich mit dem Sensor (30) gemessen wird, biologische Informationen analysiert.

7. Programm, das bewirkt, dass ein Prozessor als die Indikatorableitungseinheit (50) und die Verarbeitungseinheit (51) der Vorrichtung zur Analyse biologischer Informationen (1) nach einem der Ansprüche 1 bis 5 arbeitet.

8. Verfahren zur Analyse biologischer Informationen, umfassend:

    einen Schritt zum Umwandeln, mit einer Indikatorableitungseinheit (50), von Zeitreihendaten einer Blutdruck-Wellenform, die kontinuierlich von einem Sensor (30) gemessen wird, der am Körper eines Benutzers getragen wird und die Blutdruck-Wellenform für jeden der Herzschläge nicht invasiv messen kann, in ein Frequenzspektrum, und Ableiten eines Indikators auf Grundlage einer Stärke einer festgesetzten Frequenzkomponente im Frequenzspektrum; und
    einen Schritt zum Durchführen eines Vorgangs auf Grundlage des abgeleiteten Indikators mit einer Verarbeitungseinheit (51),
    wobei ein Indikator, der zum Auftreten einer Körperbewegung während der Messung der Blutdruck-Wellenform gehört, auf Grundlage der Stärke der festgesetzten Frequenzkomponente im Frequenzspektrum von der Indikatorableitungseinheit (50) abgeleitet wird und der Indikator für jeden von einer Vielzahl von Körperteilen von der Indikatorableitungseinheit (50) berechnet wird; und

wobei sich die zum Berechnen des Indikators eingesetzte Frequenzkomponente von Körperteil zu Körperteil unterscheidet.

**Revendications**

1. Dispositif d'analyse d'informations biologiques (1) comprenant :

une unité d'extraction d'indicateur (50) conçue pour convertir, dans un spectre de fréquences, des données de séries chronologiques d'une forme d'onde de pression artérielle mesurée en continu et de façon non effractive par un capteur (30) pour chacun des battements de cœur, et pour extraire un indicateur sur la base de l'intensité d'une composante de fréquence prescrite dans le spectre de fréquences ; et

une unité de traitement (51) conçue pour exécuter un processus sur la base de l'indicateur extrait,

dans lequel l'unité d'extraction d'indicateur (50) est conçue pour extraire un indicateur se rapportant à la survenance d'un mouvement corporel au cours de la mesure de forme d'onde de pression artérielle, sur la base de l'intensité de la composante de fréquence prescrite dans le spectre de fréquences, et pour calculer l'indicateur pour chaque partie d'une pluralité de parties du corps ; et

dans lequel la composante de fréquence utilisée pour calculer l'indicateur diffère d'une partie du corps à l'autre.

2. Dispositif d'analyse d'informations biologiques (1) selon la revendication 1,
dans lequel l'unité de traitement (51) est conçue pour exécuter un processus consistant à déterminer si un mouvement corporel est survenu ou non au cours de la mesure de forme d'onde de pression artérielle, sur la base de l'indicateur.

3. Dispositif d'analyse d'informations biologiques (1) selon la revendication 2,
dans lequel l'unité de traitement (51) est conçue pour déterminer qu'un mouvement corporel est survenu au cours de la mesure de forme d'onde de pression artérielle, lorsque l'indicateur est supérieur à un seuil.

4. Dispositif d'analyse d'informations biologiques (1) selon l'une quelconque des revendications 1 à 3, dans lequel l'unité de traitement (51) est conçue pour exécuter un processus consistant à réduire le bruit produit par un mouvement corporel dans la forme d'onde de pression artérielle, à partir des données de séries chronologiques de forme d'onde de pression artérielle, lorsque l'indicateur est supérieur au seuil.

5. Dispositif d'analyse d'informations biologiques (1) selon l'une quelconque des revendications 1 à 3, dans lequel l'unité de traitement (51) est conçue pour exécuter, lorsque l'indicateur est supérieur au seuil :

un processus consistant à couper ou réduire une composante de fréquence prescrite, incluse dans les données de séries chronologiques de forme d'onde de pression artérielle, un processus consistant à rejeter les données de séries chronologiques de forme d'onde de pression artérielle, ou un processus consistant à ajouter des informations indiquant une faible fiabilité aux données de séries chronologiques de forme d'onde de pression artérielle.

6. Système d'analyse d'informations biologiques (10) comprenant :

un capteur (30) qui est conçu pour mesurer de façon non effractive une forme d'onde de pression artérielle pour chacun des battements de cœur ; et
le dispositif d'analyse d'informations biologiques (1) selon l'une quelconque des revendications 1 à 5, le dispositif d'analyse d'informations biologiques (1) étant conçu pour analyser des informations biologiques à l'aide de données de la forme d'onde de pression artérielle mesurée en continu par le capteur (30).

7. Programme faisant fonctionner un processeur comme l'unité d'extraction d'indicateur (50) et l'unité de traitement (51) du dispositif d'analyse d'informations biologiques (1) selon l'une quelconque des revendications 1 à 5.

8. Procédé d'analyse d'informations biologiques comprenant :

une étape consistant à convertir, au moyen d'une unité d'extraction d'indicateur (50), dans un spectre de fréquences, des données de séries chronologiques d'une forme d'onde de pression artérielle mesurée en continu par un capteur (30) qui est porté sur le corps d'un utilisateur et qui peut mesurer de façon non effractive la forme d'onde de pression artérielle pour chacun des battements de cœur, et à extraire un indicateur sur la base de l'intensité d'une composante de fréquence prescrite dans le spectre de fréquences ; et

une étape consistant à exécuter un processus sur la base de l'indicateur extrait, au moyen d'une unité de traitement (51), dans lequel un indicateur se rapportant à la survenance d'un mouvement corporel au cours de la mesure de forme d'onde de pression artérielle sur la base de l'intensité de la composante de fréquence prescrite dans le spectre de fréquences est extrait par l'unité d'extraction d'indicateur (50), et l'indicateur est calculé par l'unité d'extraction d'indicateur (50) pour chaque partie d'une pluralité de parties du corps ; et

dans lequel la composante de fréquence utilisée pour calculer l'indicateur diffère d'une partie du corps à l'autre.

FIG. 1

# FIG. 2

EP 3 430 989 B1

FIG. 3

FIG. 4

FIG. 5

```
                        ┌──────────────────────────────────────────────┐
                        │                                              ▼
                   ┌──────────────┐              ┌──────────────┐
MEASUREMENT   ───► │  INDICATOR   │  INDICATOR   │ PROCESSING   │ ───►
DATA               │  EXTRACTION  │ ───────────► │    UNIT      │
                   │    UNIT      │              │              │
                   └──────────────┘              └──────────────┘
                          50                           51
```

FIG. 6

FIG. 7

```
        ( START )
            │
            ▼
┌──────────────────────┐
│  LOAD BLOOD PRESSURE │  900
│    WAVEFORM DATA     │
└──────────────────────┘
            │
            ▼
┌──────────────────────┐
│  CONVERT FREQUENCY   │  901
└──────────────────────┘
            │
            ▼
┌──────────────────────┐
│ OBTAIN SPECTRUM      │
│ STRENGTH OF FREQUENCY│  902
│ BAND CORRESPONDING   │
│ TO BODY PART         │
└──────────────────────┘
            │
            ▼
       903  ◇ SPECTRUM FREQUENCY  NO
          > THRESHOLD ?
            │ YES
            ▼
┌──────────────────────┐
│  EXECUTE PRESCRIBED  │  904
│      PROCESS         │
└──────────────────────┘
            │
            ▼
  906   ◇ COMPLETE        905
NEXT BODY  NO  FOR ALL BODY
  PART  ◀──   PARTS?
            │ YES
            ▼
┌──────────────────────┐
│  INVERSE TRANSFORM   │  907
└──────────────────────┘
            │
            ▼
        ( END )
```

90: SETTINGS TABLE

| PART | FREQ. BAND | THRESH. |
|---|---|---|
| FINGER | 3.3-3.6Hz | ・・・ |
| WRIST | ・・・ | ・・・ |
| ⋮ | ⋮ | ⋮ |

EP 3 430 989 B1

# FIG. 8

STRENGTH

FINGER MOVEMENT DURING MEASUREMENT

0  2  4  6  8  10 12 14  16 18 20

FREQUENCY [Hz]

EP 3 430 989 B1

FIG. 9

STRENGTH

RELEASE OF HELD BREATH

0  2  4  6  8  10 12 14  16  18  20

FREQUENCY [Hz]

EP 3 430 989 B1

FIG. 10

```
          ┌──────────┐
          │  START   │
          └────┬─────┘
               │
               ▼
  ┌──────────────────────────┐
  │   LOAD BLOOD PRESSURE     │  1200
  │      WAVEFORM DATA        │
  └────────────┬─────────────┘
               │
               ▼
  ┌──────────────────────────┐
  │    CONVERT FREQUENCY      │  1201
  └────────────┬─────────────┘
               │
               ▼
  ┌──────────────────────────┐
  │  CALCULATE APNEA LEVEL    │  1202
  └────────────┬─────────────┘
               │
               ▼
  ┌──────────────────────────┐
  │      OUTPUT GRAPH         │  1203
  └────────────┬─────────────┘
               │
               ▼
          ┌──────────┐
          │   END    │
          └──────────┘
```

FIG. 11A

STRENGTH

APNEA    Level=3

FREQUENCY [Hz]

FIG. 11B

2016/4/1

STRENGTH

APNEA    Level=3

FREQUENCY [Hz]

2016/4/5

STRENGTH

APNEA    Level=0

IMPROVE-MENT!

FREQUENCY [Hz]

EP 3 430 989 B1

**EP 3 430 989 B1**

**REFERENCES CITED IN THE DESCRIPTION**

**Patent documents cited in the description**

- JP 2008061824 A
- JP 2005532111 A
- US 2010228139 A1
- US 2009124914 A1
- US 2008064965 A1
- JP 2016082069 A
- JP 2016087003 A